# FASCICULE DE BREVET EUROPEEN

(11) **EP 0 713 695 B1**
(45) Date de publication et mention de la délivrance du brevet: **15.01.1997**
(21) Numéro de dépôt: 95402157.2
(22) Date de dépôt: 26.09.1995
(51) Int. Cl.: A61K 7/09, A61K 7/13

(54) **Procédé pour la déformation permanente des matières kératiniques**
Verfahren zur dauerhaften Verformung von Keratinmaterialien
Process for permanently shaping keratin materials

(30) Priorité: 24.11.1994 FR 9414109
(43) Date de publication de la demande: 29.05.1996
(73) Titulaire: L'OREAL, 75008 Paris (FR)
(72) Inventeur: Samain, Henri, F-91570 Bievres (FR)
(74) Mandataire: Tezier Herman, Béatrice

(56) Documents cités:
- EP-A- 0 022 996
- EP-A- 0 328 816
- EP-A- 0 369 356
- EP-A- 0 462 857
- WO-A-94/00099
- CH-A- 479 297
- US-A- 2 540 980
- US-A- 3 973 574
- US-A- 5 100 436
- PATENT ABSTRACTS OF JAPAN vol. 14 no. 265 (C-726) & JP-A-02 078607 (HIROSHI HOJO) 19 Mars 1990,

## Description

La présente invention concerne un nouveau procédé de traitement des matières kératiniques, en particulier des fibres kératiniques humaines telles que des cheveux, en vue d'obtenir une déformation permanente de ces dernières, en particulier sous la forme de cheveux permanentés, ledit procédé étant notamment utilisable dans le domaine des salons de coiffure, de beauté, de cosmétique et analogues, professionnels.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur (étape de réduction) puis, après avoir de préférence rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en plis.

Les compositions réductrices utilisables pour la mise en oeuvre de la première étape d'une opération de permanente contiennent généralement, à titre d'agents réducteurs, des sulfites, des bisulfites ou des thiols. Parmi ces derniers, on peut citer la cystéine et ses divers dérivés, la cystéamine et ses dérivés, l'acide thiolactique, l'acide thioglycolique ainsi que ses esters, notamment le monothioglycolate de glycérol, et le thioglycérol.

Pour réaliser l'étape de fixation susmentionnée, on fait le plus souvent appel, dans la pratique, à des compositions à base d'eau oxygénée ou de bromates alcalins.

Il est connu de l'état de la technique de réaliser des procédées de déformation permanente des cheveux en présence de sels de manganèse.

Le brevet US-A-2 540 980 décrit un traitement du cheveu avec un sel de manganèse dans un procédé de déformation permanente. Le sel de manganèse agit comme catalyseur d'oxydation avec l'oxygène de l'air. Ce procédé permet de s'affranchir d'une étape de fixation nécessitant l'utilisation d'un agent de fixation du type eau oxygénée. Ledit traitement consiste soit en un prétraitement du cheveu avec le sel de manganèse avant l'application de la composition réductrice, soit en l'application de la composition réductrice contenant un sel de manganèse.

Le brevet français FR-A-1 505 992 décrit un procédé de pré-traitement des cheveux devant subir ultérieurement une mise en plis, ledit procédé consistant en un processus chimique analogue à celui d'une permanente mais sans soumettre les cheveux à une déformation mécanique (absence de bigoudis ou de rouleaux). Les mises en plis réalisées sur des cheveux ainsi traités présentent de meilleures qualités et une meilleure tenue. L'étape de fixation dudit procédé peut être réalisée en l'absence d'agent d'oxydant, tel que l'eau oxygénée, en ajoutant dans la composition réductrice des sels de manganèse ; ceux-ci ont pour effet de catalyser l'oxydation en présence de l'oxygène de l'air. Selon les exemples 4 et 6 de ce document, une lotion catalytique contenant des sels de manganèse peut être appliquée après le rinçage suivant l'étape de réduction, dans le but de réaliser la neutralisation de la kératine; l'application d'une telle lotion permet d'éliminer toute odeur provenant du mercaptan employé lors de la phase de réduction.

La Demanderesse a constaté que la présence, même résiduelle, de sels de manganèse sur les cheveux induisait des problèmes au niveau de l'aptitude des cheveux à être par la suite correctement colorés. Ainsi, on observe pour des colorations d'oxydation appliquées sur des cheveux ainsi traités que les résultats tinctoriaux sont différents de ceux obtenus pour des cheveux permanentés sans apport de sels de manganèse : les nuances sont différentes. Le coiffeur n'aura donc pas le résultat obtenu habituellement avec des cheveux non traités. Ceci pose également un problème dans tous les cas où l'opération de coloration est conduite sur une chevelure à l'origine permanentée mais qui, entre temps, a aussi repoussé: on observe alors une différence de coloration entre les cheveux permanentés et les cheveux de repousse non permanentés.

La présente invention a pour but de résoudre les problèmes ci-dessus et de proposer un procédé de traitement permettant l'obtention d'une coloration homogène lorsqu'elle est appliquée sur des cheveux préalablement permanentés en employant des sels de manganèse.

Ainsi, à la suite d'importantes recherches menées sur la question, il a été trouvé par la Demanderesse que l'utilisation d'agents neutralisant l'activité du sel de manganèse, avant l'étape d'oxydation d'un procédé de déformation permanente, pouvait permettre de remédier avec succès aux divers inconvénients qui sont liés de manière inhérente à l'application de sels de manganèse sur les cheveux lors des opérations de permanentes.

La présente invention a donc pour objet un nouveau procédé de traitement pour la déformation permanente des matières kératiniques, en particulier des cheveux, du type comprenant une phase de réduction et une phase d'oxydation, la phase de réduction étant réalisée par l'application sur la matière kératinique à traiter d'une composition réductrice contenant au moins un thiol, la matière kératinique étant imprégnée d'au moins un sel de manganèse préalablement à/ou pendant l'étape de réduction, ledit procédé étant caractérisé par le fait que la matière kératinique est traitée, pendant ou après la phase de réduction et avant la phase d'oxydation, en présence d' au moins un agent neutralisant l'activité du sel de manganèse.

Le procédé selon l'invention présente l'avantage de pouvoir conserver l'effet désodorisant des sels de manganèse pendant la phase de réduction de la permanente.

Bien que l'exposé qui suit s'articule essentiellement autour du cas particulier du traitement du cheveu, on notera ici que le procédé selon l'invention est applicable à toute matière kératinique en général, notamment cils, moustaches, poils, laines et autres.

Selon l'invention, la matière kératinique est imprégnée d'au moins un sel de manganèse avant ou pendant la phase de réduction. L'imprégnation peut être réalisée de plusieurs manières. Le sel de manganèse peut être présent dans la composition réductrice, ou être appliqué en même temps.
Dans une autre variante, on applique sur la matière kératinique une composition dite "prélotion" contenant au moins un sel de manganèse, avant l'application de la composition réductrice, cette dernière pouvant également contenir au moins un sel de manganèse. De préférence, on laisse reposer la chevelure sur laquelle a été appliquée ladite prélotion pendant une durée comprise entre 10 secondes et 30 minutes, de préférence entre 1 et 5 minutes.

Les sels de manganèse utilisables dans le cadre de la présente invention ont un degré d'oxydation du manganèse égal ou supérieur à 2. On peut citer à titre d'exemple de manganèse le manganèse (II), le manganèse (III), le manganèse (IV), le manganèse (V) et le manganèse (VII).

Parmi les sels de manganèse utilisables, on peut notamment citer le diacétate de manganèse et ses hydrates tels que par exemple le diacétate de manganèse tétrahydrate, le dichlorure de manganèse et ses hydrates, les sulfates de manganèse, les carbonates de manganèse, les dihydrogénocarbonates de manganèse, l'acétylacétonate de manganèse, le triacétate de manganèse et ses hydrates, le tétrachlorure de manganèse.

Dans le cas où le sel de manganèse est présent dans la composition réductrice, la teneur de celui-ci, exprimée en équivalent de manganèse, est comprise entre 0,4 mg et 20 mg pour 100 g de composition réductrice, et de préférence entre 0,8 mg et 10 mg.

La prélotion peut être de type rincé, c'est à dire qu'après l'application de ladite prélotion et après avoir éventuellement laissé reposer, on rince la matière kératinique ainsi traitée puis on applique la composition réductrice.

La prélotion peut également être de type non rincé, c'est à dire qu'après l'application de ladite prélotion et après avoir éventuellement laissé reposer, on ne rince pas la matière kératinique et on applique la composition réductrice.

Dans le cas d'une prélotion de type rincé, la concentration en sel de manganèse, exprimée en équivalent de manganèse, est généralement comprise entre 0,8 mg et 80 mg, de préférence entre 1,6 mg et 64 mg, pour 100 g de ladite prélotion.

Dans le cas d'une prélotion de type non rincé, la concentration en sel de manganèse, exprimée en équivalent de manganèse, est généralement comprise entre 0,6 mg et 40 mg, de préférence entre 1,2 mg et 32 mg, pour 100 g de ladite prélotion.

Selon l'invention, la teneur en thiol dans la composition réductrice est généralement comprise entre 1 et 20 % en poids par rapport au poids total de ladite composition. Le pH de la composition réductrice est compris entre 5 et 10.

Par agent neutralisant l'activité du sel de manganèse, on entend désigner et couvrir notamment tout constituant capable d'inhiber l'activité du sel de manganèse dans les procédés de coloration d'oxydation.

A titre d'agent neutralisant l'activité du sel de manganèse utilisable selon l'invention, on peut plus particulièrement citer les complexants ayant des fonctions anioniques et amines et les zéolites.

On entend par agent complexant un composé qui est capable de chélater un sel de manganèse.

Les fonctions anioniques peuvent être des fonctions acide carboxylique, acide phosphonique ou acide sulfonique.

A titre de complexants ayant des fonctions acide carboxylique, on peut citer l'acide éthylènediamine tétracétique, le sel pentasodique de l'acide diéthylènetriamine pentacétique.

A titre de complexants ayant des fonctions acide phosphonique, on peut citer les acides alkylènediamino poly(méthylène phosphoniques) et leurs sels, et notamment l'acide éthylènediamine tétra (méthylène phosphonique) vendu sous la dénomination DEQUEST 2041 par la société MONSANTO ainsi que son sel pentasodique (DEQUEST 2046), l'acide hexaméthylènediamine tétra (méthylène phosphonique) (DEQUEST 2051) ainsi que son sel hexapotassique (DEQUEST 2054), l'acide diéthylènetriamine penta (méthylène phosphonique) (DEQUEST 2060S) ainsi que son sel heptasodique (DEQUEST 2066), l'acide 1-hydroxy éthylidène 1,1-diphosphonique (DEQUEST 2010).

On entend par zéolites tout silicoaluminate cristallin de métal alcalin, de préférence de sodium, d'origine naturelle ou synthétique.

Selon l'invention, l'agent neutralisant l'activité du sel de manganèse peut être appliqué sous forme de composition à différents moments du procédé, c'est à dire pendant ou après la phase de réduction, et avant la phase d'oxydation.

Lorsque l'agent neutralisant l'activité du sel de manganèse est appliqué après la phse de réduction, il peut être appliqué avant ou après le rinçage de la composition réductrice. Dans ce cas, lorsque l'agent neutralisant l'activité du sel de manganèse est un complexant, celui-ci est présent dans la composition appliquée à une concentration comprise entre 0,01 % et 5 % en poids par rapport au poids total de la composition le contenant. On laisse agir la composition jusqu'à l'imprégnation des cheveux, soit par exemple pendant une durée comprise entre 15 secondes et 15 minutes. Le pH de la composition est généralement compris entre 3 et 11, et de préférence entre 5 et 9.

Lorsque l'agent neutralisant l'activité du sel de manganèse est appliqué pendant la phase de réduction, celui-ci est présent dans la composition réductrice. Dans ce cas, la concentration de l'agent neutralisant est de préférence contrôlée pour ne pas perdre l'effet désodorisant du sel de manganèse: lorsque l'agent neutralisant est un complexant, celui-ci est présent dans la composition réductrice à une concentration telle que le taux de ligands du complexant par atome de manganèse est égal ou supérieur à 1 et égal ou inférieur à 5, et de préférence égal ou supérieur à 2 et égal ou inférieur à 4.

Dans le but de renforcer l'action du complexant à fonction anionique et amine sur l'activité du sel de manganèse, celui-ci peut être associé à d'autres agents complexants ne comportant pas de fonctions amines telles que les acides (poly) carboxyliques parmi lesquels on peut citer l'acide citrique et l'acide carbonique.

Les compositions utilisées selon l'invention peuvent se présenter, indépendemment l'une de l'autre, sous forme de lotion, épaissie ou non, de crème, de gel, ou de toute autre forme appropriée. Elles peuvent contenir des adjuvants connus notamment pour leur application capillaire.

Si l'on utilise dans le procédé selon l'invention une composition réductrice contenant au moins un sel de manganèse, celle-ci est de préférence conditionnée sous forme d'un dispositif à plusieurs compartiments ou "kit", le premier compartiment contenant le thiol et le second compartiment contenant le sel de manganèse, les autres ingrédients étant répartis entièrement ou en partie dans le compartiment contenant le thiol ou/et dans le compartiment renfermant le sel de manganèse. Le mélange des deux compartiments est effectué au moment de l'emploi. On peut également utiliser un seul conditionnement contenant le mélange à l'abri de l'air (faible volume d'air, conditionnement sans reprise d'air, conditionnement sous forme aérosol).

L'étape d'oxydation du procédé selon l'invention peut être réalisée par l'application d'une composition oxydante ou éventuellement en laissant agir l'oxygène de l'air.

La composition oxydante est du type couramment utilisé et contient comme agent oxydant de l'eau oxygénée, un bromate alcalin, un persel, un polythionate ou un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 10. Cette oxydation peut être effectuée immédiatement ou être différée.

L'invention a également pour objet une composition comprenant un thiol, un sel de manganèse et un agent neutralisant l'activité du sel de manganèse.

L'agent neutralisant l'activité du sel de manganèse est choisi parmi les complexants à fonctions anioniques et amines et les zéolites tels que définis ci-dessus.

En particulier, lorsque l'agent neutralisant est un complexant à fonctions anioniques et amines, sa concentration est telle que le taux de ligands du complexant par atome de manganèse est égal ou supérieur à 1 et égal ou inférieur à 5, et de préférence égal ou supérieur à 2 et égal ou inférieur à 4.

L'invention a également pour objet l'utilisation de cette composition comme réducteur dans un procédé pour la déformation permanente des matières kératiniques.

Des exemples concrets illustrant l'invention vont maintenant être donnés.

### Exemple A

On a préparé cinq compositions réductrices qui avaient les caractéristiques suivantes :

### Composition réductrice n° 1 (comparatif) :

| | |
|---|---|
| - acide thioglycolique | 9,2 g |
| - carbonate de sodium | 1 g |
| - monoéthanolamine qs | pH 8,5 |
| - mélange cocoylamidopropylbetaïne/monolaurate de glycérol (25/5) vendu sous la dénomination "TEGOBETAÏNE HS" par la société GOLDSCHMIDT à 30% de matière active | 0,3 g MA |
| - eau déminéralisée qsp | 100 g |

La composition est introduite dans un bidon aérosol en présence de 10 g de butane.

### Composition réductrice n° 2 (comparatif) :

| | |
|---|---|
| - acide thioglycolique | 9,2 g |
| - carbonate de sodium | 1 g |
| - monoéthanolamine qs | pH 8,5 |
| - acétate de manganèse tétrahydrate | 0,01 g (22 µg par g) |
| - mélange cocoylamidopropylbetaïne/monolaurate de glycérol (25/5) vendu sous la dénomination "TEGOBETAÏNE HS" par la société GOLDSCHMIDT à 30% de matière active | 0,3 g MA |
| - eau déminéralisée qsp | 100 g |

Le sel de manganèse est ajouté au dernier moment.
La composition est introduite dans un bidon aérosol en présence de 10 g de butane.

### Composition réductrice n° 3 (invention) :

| | |
|---|---|
| - acide thioglycolique | 9,2 g |
| - carbonate de sodium | 1 g |
| - monoéthanolamine qs | pH 8,5 |
| - acétate de manganèse tétrahydrate | 0,01 g (22 µg par g) |
| - mélange cocoylamidopropylbetaïne/monolaurate de glycérol (25/5) vendu sous la dénomination "TEGOBETAÏNE HS" par la société GOLDSCHMIDT à 30% de matière active | 0,3 g MA |
| - acide diéthylène triamine pentaacétique trihydrate à 40 % de matière active | 0,04 g (3,3 ligands par atome de manganèse) |
| - eau déminéralisée qs | 100 g |

Le sel de manganèse est ajouté au dernier moment.
La composition est introduite dans un bidon aérosol en présence de 10 g de butane.

### Composition réductrice n° 4 (comparatif) :

| | |
|---|---|
| - acide thioglycolique | 9,2 g |
| - carbonate de sodium | 1 g |
| - monoéthanolamine qs | pH 8,5 |
| - acétate de manganèse tétrahydrate | 0,01 g (22 µg par g) |
| - mélange cocoylamidopropylbetaïne/monolaurate de glycérol (25/5) vendu sous la dénomination "TEGOBETAÏNE HS" par la société GOLDSCHMIDT à 30% de matière active | 0,3 g MA |
| - o-phénantroline monohydrate | 0,012 g (3,3 ligands par atome de manganèse) |
| - eau déminéralisée qsp | 100 g |

L'o-phénantroline est un complexant à fonction amine.
Le sel de manganèse est ajouté au dernier moment.
La composition est introduite dans un bidon aérosol en présence de 10 g de butane.

### Composition réductrice n° 5 (comparatif) :

| | |
|---|---|
| - acide thioglycolique | 9,2 g |
| - carbonate de sodium | 1 g |
| - monoéthanolamine qs | pH 8,5 |
| - acétate de manganèse tétrahydrate | 0,01 g (22 µg par g) |
| - mélange cocoylamidopropylbetaïne/monolaurate de glycérol (25/5) vendu sous la dénomination "TEGOBETAÏNE HS" par la société GOLDSCHMIDT à 30% de matière active | 0,3 g MA |
| - acide diéthylène triamine pentaacétique trihydrate à 40 % de matière active | 0,10 g (8 ligands par atome de manganèse) |
| - eau déminéralisée qsp | 100 g |

Le sel de manganèse est ajouté au dernier moment.
La composition est introduite dans un bidon aérosol en présence de 10 g de butane.

On a effectué sur des perruques constituées de 15 g de cheveux gris européens à 70 % de blancs une permanente avec chacune des compositions réductrices selon le mode opératoire suivant : on applique sur les cheveux enroulés et humides (diamètre des rouleaux : 9 mm) la composition réductrice, puis on pose un bonnet plastique sur la chevelure et on attend 15 minutes. Puis on enlève le bonnet et l'on fait évaluer l'odeur dégagée par les perruques par un panel de 12 personnes. Les cheveux sont ensuite rincés puis on applique une composition oxydante ayant les caractéristiques suivantes :

| | |
|---|---|
| - eau oxygénée qsp | 8 V |
| - acide citrique qs | pH 3 |
| - eau déminéralisée qsp | 100 g |

On laisse agir pendant 10 minutes; on retire les rouleaux, puis on rince à l'eau et enfin on sèche les cheveux.

L'odeur dégagée par les perruques a été notée de 0 à 5, la note 0 étant attribuée lorsque l'odeur sent très mauvais et la note 5 correspondant à la perception d'aucune odeur.

Les résultats obtenus sont les suivants :

| **Perruque n°** | **Composition réductrice** | **Odeur perçue** |
|---|---|---|
| 1 | 1 | 1 |
| 2 | 2 | 3,4 |
| 3 | 3 | 3,2 |
| 4 | 4 | 3,2 |
| 5 | 5 | 1,5 |

Ensuite, on a appliqué sur chaque perruque n° 1 à 4 une composition colorante qui avait les caractéristiques suivantes :

### Composition colorante :

| | |
|---|---|
| - para phénylène diamine | 0,4 g |
| - para amino phénol | 0,24 g |
| - 1-méthyl 2-hydroxy 4-béta hydroxy éthyl aminobenzène | 1,2 g |
| - nonylphénol polyoxyéthyléné à 9 moles d'oxyde d'éthylène | 3 g |
| - alcool oléique | 18 g |
| - alcool éthylique | 9 g |
| - alcool benzylique | 11 g |
| - acide éthylène diamine tétraacétique | 0,2 g |
| - ammoniqaque à 22°Bé | 12,9 g |
| - monoéthanolamine | 6,5 g |
| - thiolactate d'ammonium à 50 % de matière active | 0,8 g |
| - 1-phényl 3-méthyl 5-pyrazolone | 0,15 g |
| - eau déminéralisée qsp | 100 g |

Au moment de l'emploi, cette composition est mélangée poids pour poids à de l'eau oxygénée 20 volumes.

On laisse agir la composition colorante pendant 30 minutes puis les cheveux sont lavés à l'aide d'un shampooing puis séchés.

Les colorations obtenues pour chaque perruque ont été évaluées par mesure des coordonées trichromatiques L, a, b des cheveux (colorimètre MINOLTA CHROMA METER 2002)

On a obtenu les résultats suivants :

| **Perruque** | **Coloration** | | |
|---|---|---|---|
| | **L** | **a** | **b** |
| 1 | 29,5 | 16 | 11,3 |
| 2 | 28,8 | 12,8 | 8,3 |
| 3 | 29 | 14,3 | 9,6 |
| 4 | 28,8 | 12,9 | 8,5 |

On constate que l'emploi d'une composition sans complexant sur la perruque 2 cause une déviation de la couleur par rapport à la coloration obtenue sur la perruque 1 : la diminution des paramètres a et b traduit une nuance différente. Cela met bien en évidence que l'emploi de sels de manganèse pendant la phase de réduction permet la diminution de l'odeur dégagée par les cheveux (notation égale à 3,4) mais pose un problème au niveau des résultats de coloration.
Lorsque l'on réalise la phase de réduction en présence d'un complexant à fonction anionique et à fonction amine et en respectant un rapport ligand/manganèse < 5 (exemple 3), on constate que l'odeur dégagée par les cheveux est réduite et que lorsque l'on effectue la coloration après la permanente, la déviation de la couleur est également réduite ( paramètres a et b de la perruque 3 plus élevés que ceux de la perruque 2). Si on utilise un complexant n'ayant que des fonctions amines (sans fonctions anioniques) (exemple 4), on constate que l'odeur dégagée par les cheveux est réduite mais la déviation de la couleur reste importante (paramètres a et b de la perruque 4 proches de ceux de la perruque 2). Enfin, si on utilise un complexant à fonction anionique et à fonction amine avec un rapport ligand/manganèse > 5 (exemple 5), on constate aucune action au niveau de l'odeur dégagée par les cheveux (notation égale à 1,5) : il n'y a pas d'effet de désodorisation.
Ainsi, seule la perruque 3 présente les meilleurs résultats de désodorisation et coloration.

### Exemple B

On a procédé comme à l'exemple A sur deux perruques (n° 6 et n° 7) en utilisant respectivement les compositions réductrices n° 1 et n° 3, à cette différence près que, sur la perruque n° 7, on a appliqué, après l'évaluation de l'odeur dégagée par les perruques et avant le rinçage de la composition réductrice, une composition dite lotion intrapermanente qui avait les caractéristiques suivantes :

### Lotion intrapermanente :

| | |
|---|---|
| - acide diéthylène triamine pentacétique trihydrate en solution aqueuse à 40 % de matière active | 2 g |
| - acide chlorhydrique qs | pH 5,5 |
| - eau déminéralisée qsp | 100 g |

On a obtenu les résultats suivants :

| **Perruque n°** | **Composition réductrice** | **Lotion intrapermanente** | **Odeur** | **Coloration** | | |
|---|---|---|---|---|---|---|
| | | | | **L** | **a** | **b** |
| 6 | 1 | non | 1 | 29,5 | 16 | 11,3 |
| 7 | 3 | oui | 3,2 | 29,5 | 15,8 | 11,1 |

Le résultat du test olfactif montre que la perruque n° 7 sent très peu au dégagement du bonnet. De plus, la perruque n°7 présente un résultat de coloration comparable à celui obtenu habituellement après des permanentes effectuées sans sel de manganèse.

### Exemple C

On a procédé comme à l'exemple B sur une perruque n° 8 en utilisant la composition réductrice n° 2.

On a procédé comme à l'exemple A sur une perruque n° 9 en utilisant la composition réductrice n° 2, à cette différence près que l'on a appliqué avant le rinçage de la composition oxydante la lotion intrapermanente de l'exemple B.

On a obtenu les résultats suivants :

| **Perruque n°** | **Composition réductrice** | **Lotion intrapermanente** | **Odeur** | **Coloration** | | |
|---|---|---|---|---|---|---|
| | | | | **L** | **a** | **b** |
| 1 | 1 | non | 1 | 29,5 | 16,0 | 11,1 |
| 8 | 2 | après réduction | 3,3 | 29,3 | 15,1 | 11,0 |
| 9 | 2 | après fixation | 3,3 | 29,0 | 12,4 | 8,4 |

Le résultat du test olfactif montre que les perruques n° 8 et 9 sentent très peu au dégagement du bonnet. Lorsque l'on effectue la coloration après la permanente, la déviation de la couleur est importante pour la perruque n° 9 (paramètres a et b de la perruque 9 plus faibles que ceux de la perruque 1) tandis qu'elle reste stationnaire pour la perruque n°8 (paramètres a et b de la perruque 8 proches de ceux de la perruque 1). Ainsi, l'application d'une lotion avec un agent complexant à fonction anionique et à fonction amine après l'étape de fixation ne permet pas de résoudre le problème au niveau des résultats de coloration tandis que l'application de la même lotion avant l'étape de fixation résout ce problème.

## Revendications

1. Procédé de traitement pour la déformation permanente des matières kératiniques, du type comprenant une phase de réduction et une phase d'oxydation, la phase de réduction étant réalisée par l'application sur la matière kératinique à traiter d'une composition réductrice contenant au moins un thiol, la matière kératinique étant imprégnée d'au moins un sel de manganèse préalablement à/ou pendant l'étape de réduction, caractérisé par le fait que la matière kératinique est traitée, pendant ou après la phase de réduction et avant la phase d'oxydation, en présence d'au moins un agent neutralisant l'activité du sel de manganèse dans les procédés de coloration d'oxydation.

2. Procédé selon la revendication 1, caractérisé par le fait que l'agent neutralisant l'activité du sel de manganèse est choisi parmi les complexants à fonctions anioniques et amines.

3. Procédé selon la revendication 2, caractérisé par le fait que lorsque la composition réductrice contient ledit complexant, celui-ci est présent dans la composition réductrice à une concentration telle que le taux de ligands du complexant par atome de manganèse est égal ou supérieur à 1 et égal ou inférieur à 5.

4. Procédé selon l'une des revendications 2 ou 3, caractérisé par le fait que lesdites fonctions anioniques sont choisies parmi les fonctions acide carboxylique, acide phosphonique, acide sulfonique.

5. Procédé selon l'une quelconque des revendications 2 à 4, caractérisé par le fait que le complexant est choisi parmi l'acide éthylènediamine tétracétique, le sel pentasodique de l'acide diéthylènetriamine pentacétique.

6. Procédé selon l'une quelconque des revendications 2 à 4, caractérisé par le fait que le complexant est un acide alkylènediamino poly(méthylène phosphonique) choisi parmi l'acide éthylènediamine tétra (méthylène phosphonique), l'acide hexaméthylènediamine tétra (méthylène phosphonique), l'acide diéthylènetriamine penta (méthylène phosphonique) et leurs sels.

7. Procédé selon la revendication 1, caractérisé par le fait que l'agent neutralisant l'activité du sel de manganèse est choisi parmi les zéolites.

8. Procédé selon l'une quelconque des revendications 1 à 2 et 4 à 7, caractérisé par le fait que l'agent neutralisant l'activité du sel de manganèse est appliquée sous forme d'une composition avant ou après le rinçage de ladite composition réductrice.

9. Procédé selon la revendication 8, caractérisé par le fait que ledit agent neutralisant est choisi parmi les complexants à fonctions anioniques et à fonctions amines et est présent à une concentration comprise entre 0,01 % et 5 % en poids par rapport au poids total de la composition.

10. Procédé selon les revendications 8 ou 9, caractérisé par le fait que l'on laisse agir ladite composition contenant l'agent neutralisant l'activité du sel de manganèse pendant une durée comprise entre 15 secondes et 15 minutes.

11. Procédé selon l'une quelconque des revendications 8 à 10, caractérisé par le fait que ladite composition contenant l'agent neutralisant l'activité du sel de manganèse a un pH compris entre 3 et 11.

12. Procédé selon la revendication 11, caractérisé par le fait que le pH de ladite composition est compris entre 5 et 9.

13. Procédé selon l'une quelconque des revendications 1 à 10, caractérisé par le fait que la composition réductrice contient au moins un agent neutralisant l'activité du sel de manganèse.

14. Procédé selon la revendication 3, caractérisé par le fait que le taux de ligands du complexants par atome de manganèse est égal ou supérieur à 2 et égal ou inférieur à 4.

15. Procédé selon l'une quelconque des revendications précédentes, caractérisé par le fait que la composition réductrice et/ou la composition contenant le sel de manganèse et/ou la composition contenant l'agent neutralisant l'activité du sel de manganèse comprend au moins un adjuvant cosmétique.

16. Procédé selon l'une quelconque des revendications précédentes, caractérisé par le fait que ladite matière kératinique consiste en des cheveux.

17. Composition caractérisée par le fait qu'elle comprend un thiol, un sel de manganèse et un agent neutralisant l'activité du sel de manganèse dans les procédés de coloration d'oxydation.

18. Composition selon la revendication 17 caractérisée par le fait que l'agent neutralisant l'activité du sel de manganèse est choisi parmi les complexants à fonctions anioniques et amines et que celui-ci est présent à une concentration telle que le taux de ligands du complexant par atome de manganèse est égal ou supérieur à 1 et égal ou inférieur à 5.

19. Composition selon la revendication 18, caractérisée par le fait que les dites fonctions anioniques sont choisies parmi les fonctions acide carboxylique, acide phosphonique, acide sulfonique.

20. Composition selon l'une des revendications 18 ou 19, caractérisée par le fait que le complexant est choisi parmi l'acide éthylènediamine tétracétique, le sel pentasodique de l'acide diéthylènetriamine pentacétique.

21. Composition selon l'une quelconque des revendications 18 à 19, caractérisée par le fait que le complexant est un acide alkylènediamino poly(méthylène phosphonique) choisi parmi l'acide éthylènediamine tétra (méthylène phosphonique), l'acide hexaméthylènediamine tétra (méthylène phosphonique), l'acide diéthylènetriamine penta (méthylène phosphonique) et leurs sels.

22. Composition selon la revendication 17, caractérisée par le fait que l'agent neutralisant l'activité du sel de manganèse est choisi parmi les zéolites.

23. Utilisation d'une composition selon l'une quelconque des revendications 17 à 22 comme réducteur pour la déformation permanente des matières kératiniques.

## Claims

1. Treatment process for the permanent reshaping of keratinous material, of the type comprising a reduction phase and an oxidation phase, the reduction phase being performed by application onto the keratinous material to be treated of a reducing composition containing at least one thiol, the keratinous material being impregnated with at least one manganese salt prior to/or during the reduction step, the said process being characterized in that the keratinous material is treated, during or after the reduction phase and before the oxidation phase, in the presence of at least one agent which neutralizes the activity of the manganese salt, in oxidation dyeing processes.

2. Process according to Claim 1, characterized in that the agent which neutralizes the activity of the manganese salt is chosen from complexing agents containing anionic and amine functions.

3. Process according to Claim 2, characterized in that when the reducing composition contains the said complexing agent, this agent is present in the reducing composition at a concentration such that the content of ligands in the complexing agent per manganese atom is equal to or greater than 1 and equal to or less than 5.

4. Process according to either of Claims 2 and 3, characterized in that the said anionic functions are chosen from carboxylic acid, phosphonic acid and sulphonic acid functions.

5. Process according to any one of Claims 2 to 4, characterized in that the complexing agent is chosen from ethylenediaminetetraacetic acid and the pentasodium salt of diethylenetriaminepentaacetic acid.

6. Process according to any one of Claims 2 to 4, characterized in that the complexing agent is an alkylenediaminopoly(methylenephosphonic) acid chosen from ethylenediaminetetra(methylenephosphonic) acid, hexamethylenediaminetetra(methylenephosphonic) acid, diethylenetriaminepenta(methylenephosphonic) acid and the salts thereof.

7. Process according to Claim 1, characterized in that the agent which neutralizes the activity of the manganese salt is chosen from zeolites.

8. Process according to any one of Claims 1 to 2 and 4 to 7, characterized in that the agent which neutralizes the activity of the manganese salt is applied in the form of a composition before or after the said reducing composition is rinsed out.

9. Process according to Claim 8, characterized in that the said neutralizing agent is chosen from complexing agents containing anionic functions and amine functions, and is present at a concentration between 0.01 % and 5 % by weight relative to the total weight of the composition.

10. Process according to Claims 8 or 9, characterized in that the said composition containing the agent which neutralizes the activity of the manganese salt is left to act for a period of between 15 seconds and 15 minutes.

11. Process according to any one of Claims 8 to 10, characterized in that the said composition containing the agent which neutralizes the activity of the manganese salt has a pH of between 3 and 11.

12. Process according to Claim 11, characterized in that the pH of the said composition is between 5 and 9.

13. Process according to any one of Claims 1 to 10, characterized in that the reducing composition contains at least one agent which neutralizes the activity of the manganese salt.

14. Process according to Claim 3, characterized in that the content of ligands in the complexing agents per manganese atom is equal to or greater than 2 and equal to or less than 4.

15. Process according to any one of the preceding claims, characterized in that the reducing composition and/or the composition containing the manganese salt and/or the composition containing the agent which neutralizes the activity of the manganese salt comprises at least one cosmetic adjuvant.

16. Process according to any one of the preceding claims, characterized in that the said keratinous material consists of hair.

17. Composition, characterized in that it comprises a thiol, a manganese salt and an agent which neutralizes the activity of the manganese salt, in oxidation dyeing processes.

18. Composition according to Claim 17, characterized in that the agent which neutralizes the activity of the manganese salt is chosen from complexing agents containing anionic and amine functions and in that it is present at a concentration such that the content of ligands in the complexing agent per manganese atom is equal to or greater than 1 and equal to or less than 5.

19. Composition according to Claim 18, characterized in that the said anionic functions are chosen from carboxylic acid, phosphonic acid and sulphonic acid functions.

20. Composition according to either of Claims 18 and 19, characterized in that the complexing agent is chosen from ethylenediaminetetraacetic acid and the pentasodium salt of diethylenetriaminepentaacetic acid.

21. Composition according to either one of Claims 18 and 19, characterized in that the complexing agent is an alkylenediaminopoly(methylenephosphonic) acid chosen from ethylenediaminetetra(methylenephosphonic) acid, hexamethylenediaminetetra(methylenephosphonic) acid, diethylenetriaminepenta(methylenephosphonic) acid and the salts thereof.

22. Composition according to Claim 17, characterized in that the agent which neutralizes the activity of the manganese salt is chosen from zeolites.

23. Use of a composition according to any one of Claims 17 to 22, as a reducing composition for the permanent reshaping of keratinous material.

## Patentansprüche

1. Behandlungsverfahren zur dauerhaften Verformung von Keratinmaterialien, das einen Reduktionsschritt und einen Oxidationsschritt umfaßt, wobei der Reduktionsschritt durch Auftragen einer reduzierenden Zusammensetzung, die mindestens ein Thiol enthält, auf das zu behandelnde Keratinmaterial durchgeführt wird, und wobei das Keratinmaterial vor dem Reduktionsschritt oder während des Reduktionsschrittes mit mindestens einem Mangansalz imprägniert wird,
dadurch gekennzeichnet, daß
das Keratinmaterial während des Reduktionsschrittes oder nach dem Reduktionsschritt und vor dem Oxidationsschritt in Gegenwart mindestens eines Mittels behandelt wird, das die Wirksamkeit des Mangansalzes in Verfahren zum oxidativen Färben aufhebt.

2. Verfahren nach Anspruch 1, dadurch gekennzeichnet, daß das Mittel, das die Wirksamkeit des Mangansalzes aufhebt, unter den Komplexbildnern mit anionischen Gruppen und Aminogruppen ausgewählt ist.

3. Verfahren nach Anspruch 2, dadurch gekennzeichnet, daß, wenn die reduzierende Zusammensetzung den Komplexbildner enthält, dieser in der reduzierenden Zusammensetzung in einer solchen Konzentration vorliegt, daß die Zahl der Liganden des Komplexbildners pro Manganatom gleich oder größer als 1 und gleich oder kleiner als 5 ist.

4. Verfahren nach Anspruch 2 oder 3, dadurch gekennzeichnet, daß die anionischen Gruppen unter Carbonsäuregruppen, Phosphonsäuregruppen und Sulfonsäuregruppen ausgewählt sind.

5. Verfahren nach einem der Ansprüche 2 bis 4, dadurch gekennzeichnet, daß der Komplexbildner unter Ethylendiamintetraessigsäure und dem Pentanatriumsalz von Diethylentriaminpentaessigsäure ausgewählt ist.

6. Verfahren nach einem der Ansprüche 2 bis 4, dadurch gekennzeichnet, daß der Komplexbildner eine Alkylendiaminpolymethylenphosphonsäure ist, die unter Ethylendiamintetramethylenphosphonsäure, Hexamethylendiamintetramethylenphosphonsäure und Diethylentriaminpentamethylenphosphonsäure sowie deren Salzen ausgewählt ist.

7. Verfahren nach Anspruch 1, dadurch gekennzeichnet, daß das Mittel, das die Wirksamkeit des Mangansalzes aufhebt, unter den Zeolithen ausgewählt ist.

8. Verfahren nach einem der Ansprüche 1, 2 und 4 bis 7, dadurch gekennzeichnet, daß das Mittel, das die Wirksamkeit des Mangansalzes aufhebt, in Form einer Zusammensetzung vor oder nach dem Ausspülen der reduzierenden Zusammensetzung aufgetragen wird.

9. Verfahren nach Anspruch 8, dadurch gekennzeichnet, daß das Mittel, das die Wirksamkeit des Mangansalzes aufhebt, unter den Komplexbildnern mit anionischen Gruppen und Aminofunktionen ausgewählt ist und in einer Konzentration im Bereich von 0,01 bis 5 Gew.%, bezogen auf das Gesamtgewicht der Zusammensetzung, vorliegt.

10. Verfahren nach einem der Ansprüche 8 oder 9, dadurch gekennzeichnet, daß man die Zusammensetzung, die das Mittel, das die Wirksamkeit des Mangansalzes aufhebt, für eine Zeitdauer von 15 Sekunden bis 15 Minuten einwirken läßt.

11. Verfahren nach einem der Ansprüche 8 bis 10, dadurch gekennzeichnet, daß die Zusammensetzung, die das Mittel enthält, das die Wirksamkeit des Mangansalzes aufhebt, einen pH-Wert im Bereich von 3 bis 11 aufweist.

12. Verfahren nach Anspruch 11, dadurch gekennzeichnet, daß der pH-Wert der Zusammensetzung im Bereich von 5 bis 9 liegt.

13. Verfahren nach einem der Ansprüche 1 bis 10, dadurch gekennzeichnet, daß die reduzierende Zusammensetzung mindestens ein Mittel enthält, daß die Wirksamkeit des Mangansalzes aufhebt.

14. Verfahren nach Anspruch 3, dadurch gekennzeichnet, daß die Zahl der Liganden des Komplexbildners pro Manganatom gleich oder größer als 2 und gleich oder kleiner als 4 ist.

15. Verfahren nach einem der vorhergehenden Ansprüche, dadurch gekennzeichnet, daß die reduzierende Zusammensetzung und/oder die Zusammensetzung, die das Mittel enthält, das die Wirksamkeit des Mangansalzes aufhebt, mindestens einen kosmetischen Hilfstoff enthält.

16. Verfahren nach einem der vorhergehenden Ansprüche, dadurch gekennzeichnet, daß das Keratinmaterial aus Haaren besteht.

17. Zusammensetzung, dadurch gekennzeichnet, daß sie ein Thiol, ein Mangansalz und ein Mittel enthält, das die Wirksamkeit des Mangansalzes in Verfahren zum oxidativen Färben aufhebt.

18. Zusammensetzung nach Anspruch 17, dadurch gekennzeichnet, daß das Mittel, das die Wirksamkeit des Mangansalzes aufhebt, unter den Komplexbildnern mit anionischen Gruppen und Aminofunktionen ausgewählt ist und in einer solchen Konzentration vorliegt, daß die Zahl der Liganden des Komplexbildners pro Manganatom gleich oder größer als 1 und gleich oder kleiner als 5 ist.

19. Zusammensetzung nach Anspruch 18, dadurch gekennzeichnet, daß die anionischen Gruppen unter Carbonsäuregruppen, Phosphonsäuregruppen und Sulfonsäuregruppen ausgewählt sind.

20. Zusammensetzung nach einem der Ansprüche 18 oder 19, dadurch gekennzeichnet, daß der Komplexbildner unter Ethylendiamintetraessigsäure und dem Pentanatriumsalz von Diethylentriaminpentaessigsäure ausgewählt ist.

21. Zusammensetzung nach einem der Ansprüche 18 oder 19, dadurch gekennzeichnet, daß der Komplexbildner eine Alkylendiaminpolymethylenphosphonsäure ist, die unter Etyhlendiamintetramethylenphosphonsäure, Hexamethylendiamintetramethylenphosphonsäure und Diethylentriaminpentamethylenphosphonsäure sowie deren Salzen ausgewählt ist.

22. Zusammensetzung nach Anspruch 17, dadurch gekennzeichnet, daß das Mittel, das die Wirksamkeit des Mangansalzes aufhebt, unter den Zeolithen ausgewählt ist.

23. Verwendung einer Zusammensetzung nach einem der Ansprüche 17 bis 22 als Reduktionsmittel für die dauerhafte Verformung von Keratinmaterialien.
